(19) Europäisches Patentamt
European Patent Office
Office européen des brevets

(11) EP 2 529 737 A1

(12) EUROPEAN PATENT APPLICATION

(43) Date of publication:
**05.12.2012 Bulletin 2012/49**

(21) Application number: **12002256.1**

(22) Date of filing: **18.07.2008**

(51) Int Cl.:
***A61K 31/415*** $^{(2006.01)}$ ***A61K 31/145*** $^{(2006.01)}$
***A61P 31/04*** $^{(2006.01)}$

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **20.07.2007 GB 0714226**
**07.09.2007 US 967801 P**

(62) Document number(s) of the earlier application(s) in accordance with Art. 76 EPC:
**08776019.5 / 2 180 900**

(27) Previously filed application:
**18.07.2008 PCT/GB2008/002499**

(71) Applicant: **E-Therapeutics plc**
**Newcastle Upon Tyne**
**NE2 4LZ (GB)**

(72) Inventors:
- **Young, Malcom Philip**
  **Newcastle upon Tyne NE2 4LZ (GB)**
- **Yates, Catherine Mary**
  **Sutton Coldfield**
  **B73 6EE (GB)**
- **Idowu, Olusola Clement**
  **Bensham**
  **NE8 1UY (GB)**
- **Charlton, Julie Anne**
  **West Woodburn**
  **Hexham**
  **NE48 2TU (GB)**

(74) Representative: **Gilholm, Stephen Philip**
**IPheions Intellectual Property**
**Buzzard Office**
**The Hawk Creative Business Park**
**Easingwold,**
**York YO61 3FE (GB)**

Remarks:
This application was filed on 29-03-2012 as a divisional application to the application mentioned under INID code 62.

(54) **Antibacterial combination therapy**

(57) There is described a composition comprising clotrimazole, or a salt thereof, and disulfiram, or a derivative thereof, for treating an infection contributed to or caused by multi-drug resistant bacterial species.



Printed by Jouve, 75001 PARIS (FR)

## Description

## FIELD OF THE INVENTION

The present invention provides a product comprising a synergistic combination of disulfiram, or derivative or metabolite thereof, and a therapeutically active imidazole, as a combined preparation for the treatment of infections caused or contributed to by multi-drug resistant bacterial species.

BACKGROUND

Micro-organisms that have developed or acquired ways of overcoming the inhibitory effects of antibiotics have been selected through the prolific use of these drugs both in human medicine and animal husbandry, indiscriminate prescribing practices, and patient non-compliance with treatment regimes. Therapeutic options for the treatment of such drug-resistant microorganisms, especially bacteria, are becoming increasingly limited. The problem of antibiotic resistance is exacerbated by the spread of drug-resistant organisms, and the dissemination of resistance genes between bacteria. The threat to the successful management of bacterial infections posed by the development and spread of antibiotic resistance is one of the most significant problems within healthcare and veterinary medicine.

*Staphylococcus aureus* is a major cause of serious healthcare associated infection (HAI). In the USA, over 50% of clinical *S. aureus* isolates are resistant to the β-lactam methicillin (NNIS, 2004). Similarly, reports of methicillin-resistant *S. aureus* (MRSA) in animals have become more frequent in recent years (O'Mahony *et al.,* 2005); MRSA has been isolated from dogs, cats, cattle, sheep, chickens, rabbits and horses (Devriese and Hommez, 1975, Hartmann *et al.,* 1997, Pak *et al.,* 1999, Tornlin *et al.,* 1999, Lee, 2003, Goni *et al.,* 2004, and Weese 2004).

In both human and veterinary medicine, bacterial biofilms (structured communities of bacterial cells enclosed in a self-produced polymeric matrix and adherent to an inert or living surface (Costerton *et al.,* 1999)) are a significant problem. In animal husbandry, bacterial biofilms can develop on poultry processing instrumentation (Amold & Silvers, 2000) and may cause treatment failure of mastitis in cows infected with S. aureus (Melchior *et al.,* 2006). In human healtheare, biofilms of bacteria, have been shown to colonise many medical devices, including orthopaedic implants (Bahna *et al.,* 2007). In the UK, 35% of hip prostheses' infection is attributable to *S. aureus,* resulting in septic loosening, fracture non-union and osteomyelitis (Sanderson, 1991). The association of MRSA with the use of orthopaedic devices is extremely problematic due to the increased spectrum of resistance of this organism, in addition to the protection from the immune system given by the biofilm growth phase, often necessitating the removal of a contaminated device, causing further trauma to the patient and increasing medical costs. Colonisation with MRSA is the general precursor to the development of an MRSA infection, so interventions that reduce levels of human colonisation or the colonisation of surfaces such as medical devices will reduce the spread of infections in healthcare facilities.

Methicillin resistance in *S. aureus* develops by the alteration of the target of the drug. β-lactam antibiotics, such as methicillin, act on sensitive strains by binding to and inhibiting proteins called "Penicillin Binding Proteins". Resistance to methicillin in *S. aureus* occurs by the alteration one of these proteins, PBP2', so that β-lactams bind poorly to it. This results in the bacterium becoming resistant to all currently available β-lactam drugs. MRSA infections can be treated with glycopeptide drugs, such as vancomycin. The rise in prevalence of MRSA and emerging high levels of resistance to antinoglycosides and ampicillin in Enterococci, have resulted in an increased reliance on vancomycin. This has driven the subsequent emergence of vancomycin resistant pathogens. Of particular note are strains commonly known as vancomycin intermediately sensitive *Staphylococcus aureus* (VISA) and vancomycin resistant *Staphylococcus aureus* (VRSA), all of which are multi-drug resistant. The emergence of VISA and VRSA means that current antibiotics may become ineffective for the treatment of human infections such as endocarditis, bacteraemia and osteomyelitis.

The administration of vancomycin to patients with recurrent MRSA infections causes an increased risk of the emergence of VISA or VRSA strains. The vast majority of VISA infections in the USA occur in patients with recurrent MRSA treated with vancomycin (Appelbaum, 2006). Although a dramatic reduction in the use of glycopeptides such as vancomycin would reduce the emergence and spread of VISA and VRSA, this is not practical without the use of alternative compounds that do not promote the emergence of multiple resistance.

VISA and VRSA infections are associated with vancomycin treatment of MRSA infections, but are genotypically and phenotypically distinct from these and other vancomycin sensitive gram-positive bacteria, tending to form discrete clonal lineages. The acquisition of mobile genetic elements carrying resistance genes and often virulence determinants results in strains that are often resistant to a number of drugs. Resistance can be specific, i.e. particular to a certain drug or class of drugs, or nonspecific in that the resistance applies to a range of drugs, not necessarily related. In the case of VISA, an increase in cell wall thickness is a major contributor to the observed drug resistance.

VISA and VRSA may be defined as any staphylococcal strain with a vancomycin MIC of 4-8mg/L (VISA) or greater or equal to 8mg/L (VRSA). These levels of resistance can be due to an increase in cell wall thickness, by the production of cell-wall precursors incapable of binding vancomycin, or via another mechanism. Susceptible gram-positive

organisms synthesise cell wall precursors ending in D-ala-D-ala, whereas vancomycin resistant gram-positive organisms synthesise, for example, D-ala-D-lac precursors. The presence of vancomycin resistance in staphylococcal strains may be identified by the measurement of the MIC to vancomycin by broth or agar dilution, or by Etest®, or by the identification of *vanA, vanB, vanC, vanD, vanE, vanG* genes, or similar, by polymerases chain reaction (PCR). The current invention also encompasses the subclass of VISA strains that are heterogeneous VISA (hVISA); these are vancomycin susceptible methicillin-resistant *Staphylococcus aureus* by conventional testing but have a sub-population of intermediately resistant cells. hVISA strains are thought to be the precursors of VISA.

The management of human infections caused by VISA and VRSA reflect these genotypic and phenotypic differences, and require greater investment in hospital infrastructure, facilities for patient isolation, and infection control measures than for other strains of Staphylococci.

The treatment options for infections contributed to or caused by VISA or VRSA are now severely limited. There is an urgent need to discover new compounds that inhibit or kill such organisms, and to limit the development and spread of these multiply-resistant pathogens.

It has been found that certain imidazoles and or their derivatives are capable of inhibiting the growth of MRSA (Lee & Kim, 1999) and/or VISA and VRSA (UK patent applications P14994GB and P148124GB). Similarly, there is evidence that disulfiram and its major *in vivo* metabolites diethyldithiocarbarnate (DDTC), and dimethyldithiocarbamate (DMTC), possess some antibacterial activity against MRSA, with DMTC demonstrating *in vitro* synergy against some gram-positive bacteria with the aminoglycoside antibiotic gentamicin (Taylor *et al.,* 1987).

Synergy between antibiotics may occur when two antibiotics target bacterial proteins within the same metabolic pathway. Trimethoprim and sulphamethoxazole are commonly administered together as co-trimoxazole because they target two different enzymes in the bacterial folic acid synthesis pathway. Synergy may also occur when a resistance mechanism, such as an efflux pump, is inhibited, permitting the accumulation of an antibiotic that if administered singly, may be removed by the efflux pump. It is impossible to predict that two compounds will act synergistically to give an antibacterial effect greater then the sum of the effects of the individual drugs, unless the mechanism of action of each agent is known. Similarly, if a compound acts synergistically with a particular antibiotic, it cannot be predicted that a combination with an antibiotic acting on different bacterial targets or on different bacterial strains will also exhibit synergy.

The present invention discloses the knowledge that the combination of certain imidazoles with either disulfiram, DDTC or DMTC is not only capable of inhibiting the growth of MRSA, but additionally, through specific synergy against VISA/VRSA strains (but not MRSA strains), limits the development of VISA and VRSA from MRSA, thus limiting the development and spread of these more dangerous pathogens,

An objective of the present invention is to provide a new and effective treatment for infections contributed to or caused by MRSA, VISA or VRSA, that is capable of reducing the likelihood of the development and/or spread of VISA and VRSA.

## SUMMARY OF THE INVENTION

In a first aspect, the present invention provides a composition comprising a therapeutically active imidazole, and/or a derivative thereof, and disulfiram, or a derivative thereof.

In particular, the present invention provides a composition as hereinbefore described for treating an infection contributed to or caused by multi-drug resistant bacterial species.

Such multi-drug resistant bacterial species include, but shall not be limited to, MRSA, VISA, and/or VRSA.

Furthermore, and in a second aspect, the present invention provides a method of treating a subject suffering from an infection contributed to or caused by multi-drug resistant bacterial species, said method comprising the step of administering an effective amount of a therapeutically active imidazole, and/or a derivative thereof, with disulfiram, or a derivative or a metabolite thereof, separately, simultaneously or sequentially.

The method of the invention particularly provides a method of treating a subject suffering from an infection contributed to or caused by one or more of MRSA, VISA and VRSA.

In particular, the present invention concerns the use of a compound comprising imidazole and/or derivatives thereof, in combination with disulfiram or a derivative/metabolite thereof, for the preparation of medicaments or for use in methods effective in treating infections contributed to or caused by MRSA, VISA or VRSA.

Exemplary compounds comprising a therapeutically effective imidazole for use in connection with the present invention are provided by the following general formula

(Formula I):

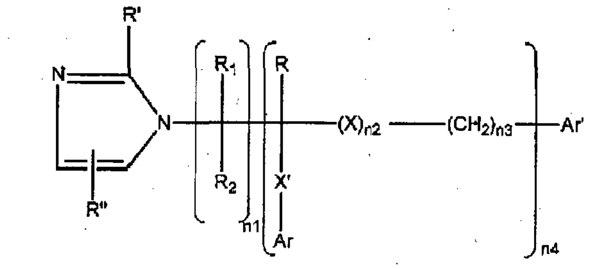

Formula I

Wherein:

$R_1$ and $R_2$ are independently selected from hydrogen lower alkyl, phenyl or substituted phenyl, wherein said substituted phenyl contains at least one phenyl substituent selected from the group consisting of halo, lower alkyl and lower alkoxy;
R is independently selected from hydrogen, lower alkyl, phenyl or substituted phenyl wherein said substituted phenyl contains at least one phenyl substituent selected from the group consisting of halo, lower alkyl and lower alkoxy;
$n_1$ is zero or 1;
X is oxy, S, NH or

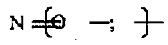

$n_2$ is zero or 1;
$n_3$ is zero, 1 or 2
X' is S, Oxy or not present
Ar is independently selected from the group consisting of phenyl, substituted phenyl,
thienyl and halothienyl, said substituted phenyl containing at least one phenyl substituent selected from the group consisting of halo, lower alkyl and lower alkoxy;$n_4$ is zero or 1
Ar' is a member selected from the group consisting of phenyl, substituted phenyl and
α-tetralyl, said substituted phenyl containing at least one phenyl substituent selected from the group consisting of phenyl, thienyl, phenyl thio, halo, lower alkyl, lower alkoxy, cyano, nitro and amino;
R' is a member selected from the group consisting of hydrogen, methyl and ethyl; and
R" is a member selected from the group consisting of hydrogen and methyl;
provided that:

(i) when X is NH, then said R is hydrogen;
(ii) when said Ar' is a substituted phenyl containing at least one phenyl substituent selected from the group consisting of nitro and amino, then said X is oxy and said $n_3$ is zero;
(iii) when said Ar' is α-tetralyl, then said X is NH and said $n_3$ is zero; and
(iv) when X is oxy and said Ar' is a member selected from the group consisting of phenyl and substituted phenyl containing at least one phenyl substituent selected from the group consisting of halo, lower alkyl, lower alkoxy and cyano, then said $n_3$ is other than zero.

It is to be understood that the terms "lower alkyl" and "lower alkoxy" encompass straight or branch chained hydrocarbons having from about 1 to about 6 carbons, such as, for example, methyl, ethyl, propyl, isopropyl, butyl, pentyl, hexyl and the like alkyls, and, respectively, the corresponding alkoxys such as methoxy, ethoxy, propoxy, isopropoxy, etc.

The preferred lower alkyl and lower alkoxy are methyl and methoxy, respectively. Furthermore, the term "halo" refers to halogens of atomic weight less than 127, i.e., fluoro, iodo, bromo and chloro. Preferred substituted phenyls with respect to the symbol Ar are mono-, di- and trihalo-phenyl, thiol-dihalo-phenyl, lower alkylphenyl and lower alkoxy phenyl; and mono-, di- and tri-halophenyl, di-phenyl, thiol-phenyl, lower alkoxyphenyl, cyanophenyl, mono-, di-nitrophenyl and amino phenyl with regard to the symbol Ar'.

Of particular interest are the therapeutically active imidazoles selected from the group consisting of clotrimazole, econazole, miconazole, butoconazole, fenticonazole, oxiconazole nitrate, sertaconazole, sulconazole, and tioconazole and derivatives thereof.

Especially of interest are the therapeutically active imidazoles selected from the group 1-[(2-Chlorophenyl) diphenylmethyl]-1H-imidazole ($C_{22}H_{17}ClN_2$), 1-[2-[(4-Chlor phenyl)methoxy]-2-(2,4-dichloropheny)ethy]-1H-imidazole ($C_{18}H_{15}Cl_3N_2O$) and 1-[2-(2,4-Dichlorophenyl)-2-[(2,4-dichlorophenyl) methoxy]ethyl]-1H-imidazole ($C_{18}H_{14}Cl_4N_2O$), otherwise known as clotrimazole, econazole and miconazole, respectively. The formulae for each of these compounds are as follows.

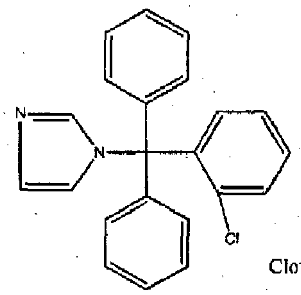

Clotrimazole

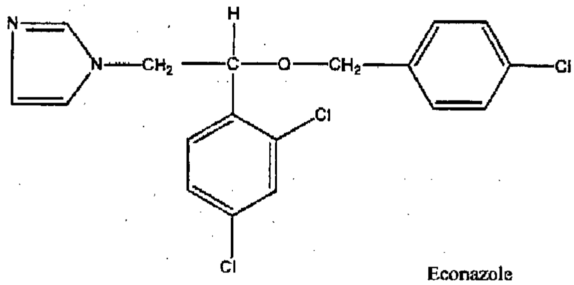

Econazole

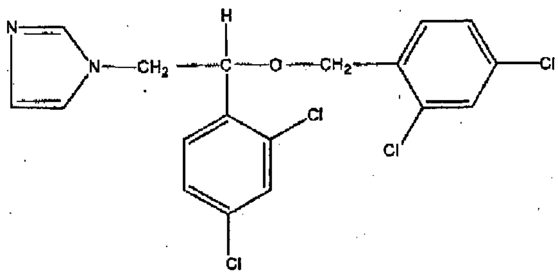

Miconazole

Other compounds of interest may include (±)-1-[4-(4-Chlorophenyl)-2[(2,6-dichlorophenyl)thio]butyl]-1H-imidazole ($C_{19}H_{17}Cl_3N_2S$: Butoconazole), 1-[2-(2,4-Dichlorophenyl)-2-[[4-phenylthio)phenyl]methoxy]ethyl]-1H-imidazole ($C_{24}H_{20}Cl_2N_2OS$: Fenticonazole), (Z)-1-(2,4-Dichlorophenyl)-2-(1H-imidazol-1-yl)ethanone O-[2,4-dichlorophenyl)-methyl]oxime mononitrate ($C_{18}H_{14}Cl_4N_4O_4$: Oxiconazole Nitrate), 1-[2-[(7-chlorobenzothiophen-3-yl)methoxy]-2-(2,4-dichlorophenyl)-ethyl]imidazole ($C_{20}H_{15}Cl_3N_2OS$: Sertaconazole), 1-[2-[[(4-chlorophenyl)methyl]-thio]-2-(2,4-dichlorophenyl)ethyl]-1H-imidazole ($C_{18}H_{15}Cl_3N_2S$: Sulconazole) and 1-[2-[(2-Chloro-3-thienyl)methoxy]-2-(2,4-dichlorophenylethyl]-1H-imidazole ($C_{16}H_{13}Cl_3N_2OS$: Tioconazole).

In addition, the present invention also encompasses uses of various salts and therapeutically active addition salts of compounds corresponding to formula (I) and derived from the abovementioned compounds comprising imidazole; in particular, for example, miconazole nitrate ($C_{18}H_{14}Cl_4N_2O \cdot HNO_3$), and econazole nitrate ($C_{18}H_{15}Cl_3N_2O \cdot HNO_3$).

Derivatives of disulfiram as hereinbefore described, shall include, but shall not be limited to, metabolites of disulfiram. Thus, compounds comprising disulfiram and/or its metabolites or derivatives for use in combination with the above imidazoles in connection with the present invention are provided by the following formulae:

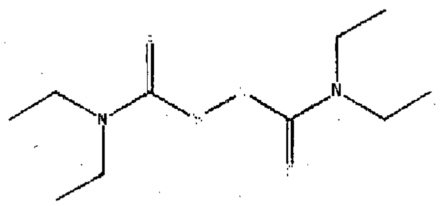

Disulfiram

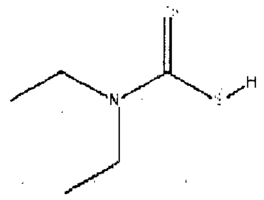

Diethyldithiocarbamate

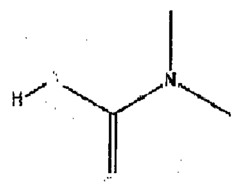

Dimethyldithiocarbamate

It may be convenient or desirable to prepare, purify, and/or handle a corresponding solvate of the compounds described herein, which may be used in any one of the uses/methods described. The term solvate is used herein to refer to a complex of solute, such as a compound or salt of the compound, and a solvent. If the solvent is water, the solvate may be termed a hydrate, for example a mono-hydrate, di-hydrate, tri-hydrate etc, depending on the number of water molecules present per molecule of substrate.

Accordingly, and in one embodiment, the present invention provides a composition comprising one or more of clotrimazole, clotrimazole nitrate, econazole, econazole nitrate, miconazole, miconazole nitrate in combination with disulfiram and/or diethyldithiocarbamate and/or dimethyldithiocarbamate in the manufacture of a medicament for the treatment of an infection contributed to or caused by MRSA, VISA, or VRSA.

In addition, we provide the use of a therapeutically active imidazole in the manufacture of a combination medicament for treating an infection, e.g. an infection contributed to or caused by MRSA, VISA, or VRSA thereby reducing the emergence of VISA or VRSA.

We further provide the use of a disulfiram in the manufacture of a combination medicament with a therapeutically active imidazole for treating an infection e.g. an infection contributed to or caused by MRSA, VISA, or VRSA thereby reducing the emergence of VISA or VRSA.

We also provide a therapeutically active imidazole in a combination medicament, e.g. with disulfiram, for treating an infection, e.g. an infection contributed to or caused by MRSA, VISA, or VRSA thereby reducing the emergence of VISA or VRSA.

We also provide disulfiram in a combination medicament, e.g. with a therapeutically active imidazole, for treating an infection e.g. an infection contributed to or caused by MRSA, VISA, or VRSA thereby reducing the emergence of VISA or VRSA.

Advantageously, synergistic combinations comprising imidazole or derivatives thereof with disulfiram and/or derivatives or metabolites thereof may be administered orally, topically to the site of an infection, transmucosally, transdermally or intravenously. Accordingly, synergistic combinations comprising imidazole or derivatives thereof with disulfiram and/or derivatives or metabolites thereof may be formulated as polymeric nanoparticles such as alginate or polylactide-co-glycolide nanoparticles, or as sterile pharmaceutical compositions comprising a pharmaceutically acceptable carrier or excipient. Such carriers or excipients are well known to one of skill in the art and may include, for example, water, saline, phosphate buffered saline, dextrose, glycerol, ethanol, ion exchangers, alumina, aluminium stearate, lecithin, serum proteins, such as serum albumin, buffer substances such as phosphates, glycine, sorbic acid, potassium sorbate, partial glyceride mixtures of saturated vegetable fatty acids, lactic acid, water salts or electrolytes, such as protamine sulphate, disodium hydrogen phosphate, potassium hydrogen phosphate, sodium chloride, zinc salts, colloidal silica, magnesium trisilicate, polyvinyl pyrrolidone, cyclodextrins, such as αcyclodextrin, βcyclodextrin, sulfobutylether$_7$-βcyclodextrin and hydroxypropyl-β-cyclodextrin, cellulose-based substances, polyethylene glycol, sodium carboxymethylcellulose, polyacrylates, waxes, polyethylene-polypropylene-block polymers, polyethylene glycol and wool fat and the

like, or combinations thereof.

Synergistic combinations comprising imidazole or derivatives thereof with disulfiram and/or derivatives or metabolites thereof may be administered in combination with another treatment. For example, synergistic combinations comprising imidazole or derivatives thereof with disulfiram and/or derivatives or metabolites thereof may be administered in combination with a chemotherapeutic agent, a detergent to facilitate permeation, an immunostimulatory compound or drug, an oligonucleotide, a cytokine, hormone or the like.

It may be possible to administer a synergistic combinations comprising imidazole or derivatives thereof with disulfiram and/or derivatives or metabolites thereof, or any combined regime as described above, transdermally via, for example, some form of transdermal delivery device. Such devices arc advantageous, particularly for the administration of antibiotic compounds, as they may allow a prolonged period of treatment relative to, for example, an oral or intravenous medicament.

Examples of transdermal delivery devices may include, for example, a patch, dressing, bandage or plaster adapted to release a compound or substance through the skin of a patient. A person of skill in the art would be familiar with the materials and techniques which may be used to transdermally deliver a compound or substance and exemplary transdermal delivery devices are provided by GB2185187, US3,249,109, U53,598,122, US4,144,317, US4,262,003 and US4,307,717.

By way of example, synergistic combinations comprising imidazole or derivatives thereof with disulfiram and/or derivatives or metabolites thereof, may be combined with some form of matrix or substrate, such as a non-aqueous polymeric carrier, to render it suitable for use in a transdermal delivery system. This mixture may be further strengthened by the use of a woven or knit, non-woven, relatively open mesh fabric, to produce a patch, bandage, plaster or the like which may be temporarily attached to a particular region of a patient's body. In this way, while in contact with a patient's skin, the transdermal delivery device releases the compound or substance directly to the site of infection or through the skin as required,

The compounds provided herein may also be used as sterilising or cleaning aids for use, for example, on surfaces to reduce and/or eliminate contamination by MRSA, VISA or VRSA. By way of example, synergistic combinations comprising imidazole or derivatives thereof with disulfiram and/or derivatives or metabolites thereof such as, for example miconazole or miconazole nitrate with disulfiram, may be prepared for application to any surface suspected of being contaminated by MRSA, VISA or VRSA. For example, compounds of the present invention may be added to or diluted in an appropriate excipient or solution prior to use as a sterilising or Cleaning agent. Exemplary excipients arc described above. Such sterilising or cleaning solutions may be used to decontaminate, for example, furniture, floors, equipment including for example specialised hospital equipment and/or surgical equipment

Advantageously, synergistic combinations comprising imidazole or derivatives thereof with disulfiram and/or derivatives or metabolites thereof, may be administered to a medical or veterinary surface to inhibit the growth of MRSA, VISA and VRSA, and reduce the likelihood of the emergence and spread of vancomycin resistance in that environment. The term "surface" used herein, refers to any surface whether medical or industrial, that provides an interface between a fluid and a solid. The interface between fluid and solid may be intermittent, and may be caused by flowing or stagnant fluid, aerosols, or other means for air-borne fluid exposure. The surface described herein, refers more specifically to a plane whose mechanical structure is compatible with the adherence of bacteria such as S. *aureus.* In the context of the current patent, the terminology "medical or veterinary surface" encompasses the inner and outer aspects of various instruments and devices, both disposable and non-disposable. Examples include the entire spectrum of medical devices

As used herein, the terminology "surfaces found in medical environments" includes the inner and outer aspects of various instruments and devices, whether disposable or intended for repeated uses. Examples include the entire spectrum of articles adapted for medical use, including scalpels, needles, scissors and other devices used in invasive surgical, therapeutic or diagnostic procedures; implantable medical devices, including artificial blood vessels, catheters and other devices for the removal or delivery of fluids to patients, artificial hearts, artificial kidneys, orthopaedic pins, plates and implants; catheters and other tubes (including urological and biliary tubes, endotracheal tubes, peripherally insertable central venous catheters, dialysis catheters, long term tunnelled central venous catheters, peripheral venous catheters, short term central venous catheters, arterial catheters, pulmonary catheters. Swan-Ganz catheters, urinary catheters, peritoneal catheters), urinary devices (including long term urinary devices, tissue bonding urinary devices, artificial urinary sphincters, urinary dilators), shunts (including ventricular or arterio-venous shunis); prostheses (including breast implants, penile prostheses, vascular grafting prostheses, heart valves, artificial joints, artificial larynxes, otological implants), vascular catheter ports, wound drain tubes, hydrocephalus shunts, pacemakers and implantable defibrillators, and the like. Other examples will be readily apparent to practitioners in these arts. Surfaces found in the medical environment also include the inner and outer aspects of pieces of medical equipment, medical gear worn or carried by personnel in the health care setting. Such surfaces can include counter tops and fixtures in areas used for medical procedures or for preparing medical apparatus, tubes and canisters used in respiratory treatments, including the administration of oxygen, of solubilised drugs in nebulisers and of aesthetic agents, Also included are those surfaces intended as biological barriers to infectious organisms in medical settings, such as gloves, aprons and face-shields. Commonly

used materials for biological barriers may be latex-based or non-latex based, such as vinyl. Other such surfaces can include handles and cables for medical or dental equipment not intended to be sterile. Additionally, such surfaces can include those non-sterile external surfaces of tubes and other apparatus found in areas where blood or body fluids or other hazardous biomaterials arc commonly encountered.

In a further embodiment, the compounds describe herein may be used to eliminate and/or reduce contamination by MRSA, VISA or VRSA on parts of the body, particularly for example, the hands. Synergistic combinations comprising imidazole or derivatives thereof with disulfiram and/or derivatives or metabolites thereof, may be diluted as an aqueous or non-aqueous solution (dissolved in aqueous, non aqueous or organic solvent) and which may be applied to a body part, for example the hands, Such a solution may find particular application in, for example hospitals, care homes and or nurseries where the prevalence and transmission rates of MRSA, VISA or VRSA are often high.

In a further embodiment, the methods and medicaments described herein may be used prophylactically as a means to prevent the development of an infection caused or contributed to by MRSA, VISA or VRSA, or to reduce the likelihood of the development of VISA or VRSA from an MRSA infection. Medicaments and/or methods for prophylactic use may be administered or applied to any person at risk of developing an infection caused or contributed to by MRSA, VISA or VRSA. For example, people working in care homes, nursing homes, sports centres, community centres, shops, restaurants, cafes, nurseries and/or schools may require prophylactic treatments.

Advantageously, the medicaments and/or methods described herein may have particular application in institutions housing, sheltering, caring or otherwise holding people or patients vulnerable to or "at risk" of developing or contracting MRSA, VISA or VRSA. The medicaments and methods may be particularly useful in hospitals, nursing homes, nurseries and/or schools. More generally, an elderly, young or immunocompromised person or patient may particularly benefit from the medicaments and methods described herein. Moreover, the methods and medicaments of the present invention may be particularly useful to those undergoing a prolonged stay in hospital, for example in an intensive care facility.

Additionally, or alternatively, the medicaments and methods described herein may be useful in community centres, sports facilities, shops, restaurants, cafes or other places where transmission of bacteria, particularly MRSA, VISA or VRSA, is likely.

**DETAILED** DESCRIPTION

**METHODS:**

In example experiments miconazole nitrate, econazole nitrate, clotrimazole, and disulfiram or DDS were dissolved in DMSO. Experiments were repeated with the disulfiram or DDS dissolved in ethanol. Other solvents that may be used include caster oil, pyridine, and 0.9% saline. For IV administration agents may be solubilised in polyethoxylated caster oil, or cyclodextrins such as sulfobutylether$_7$-βcyclodextrin or hydroxypropyl-β-cyclodextrin and lactic acid. Minimum inhibitory concentrations (MICs) of a range of clinical and control bacterial organisms were measured according to BSAC (British Society for Antimicrobial Chemotherapy) guidelines (Andrews 2001), for single agents and combinations of agents, described briefly as follows. MICs were measured by agar dilution (Experiment A) and by broth microdilution (experiment B).

**PREPARATION** OF AGAR PLATES AND **BROTHS.**

Stock solutions of each agent were prepared using the formula:

$$\frac{1000}{P} \times V \times C = W$$

Where P = μg of active compound per mg (μg/mg)

V = volume required (mL)
C = final concentration of solution (mg/L)
W= weight of agent (mg) to be dissolved in volume V (mL)

Stock solutions were prepared at concentrations of 1000mg/L and 100mg/L. The appropriate amounts of each stock solution were added to separate Petri dishes give a range of final concentrations (after the addition of 20mL molten agar): from 0.12 to 32 mg/L. In experiment A, a 1:1 ratio of each agent was added to the appropriate Petri dish so that a 2mg/L plate contained 2mg/L of each agent. Volumes (20mL) of cooled molten IST agar (oxoid) was added to each

Petri dish and mixed by swirling. After drying, the plates were stored at 4°C and protected from light. Plates were used on the day of preparation. In experiment B, a 1:1 ratio of drugs was made up first, and then, for example, the appropriate volume to give 2mg/L of this mixed solution was added to the 2mg/L well of a microtitre plate, After which, the appropriate volume of broth was added to give a final concentration in the well of 2mg/L total drug.

**PREPARATION OF INOCULUM**

The test organisms were grown overnight in 5mL IST broth. Using a dilution in 0.9% saline of 1:500 for Gram-negative organisms and 1:100 for Gram-positive organisms, the appropriate agar plates (experiment A) were inoculated using a multipoint inoculator. For the broth microdilution, each well of a microtitre plate was inoculated with diluted overnight culture to give a final inoculum of 5 x 10 cfu/mL.

**INCUBATION**

Agar plates and microtitre plates were incubated at 37°C in air for 18-20 hours.

**INTERPRETATION OF RESULTS**

The MIC is the minimum amount of an antibiotic at which there is no visible growth of bacteria. Tiny single colonies or faint hazes were not counted as growth, Synergy was reported if the inhibitory effect of the drugs in combination were greater than the sum of the inhibitory effects of each drug singly.

**RESULTS:**

Table one shows that the combination of disulfiram (dissolved in ethanol) with miconazole gives substantially lower MICs than the comparative MTCs for miconazole and disulfiram used singly. Although lower MICs are observed with the combination against all MRSA and VISA strains tested, the synergistic effect appears to be particularly strong for bacterial strains with reduced susceptibility to vancomycin. The ethanol control demonstrates that this solute did not affect the measurement of the MICs. When the solvent DMSO was used to solubilise the disulfiram, similar results were obtained.

**Table 1.** Results of experiment 1 (agar dilution), comparing the effect of disulfiram and miconazole used in combination with the activity of these drugs used singly.

| Strain | Type of strain | Miconazole | Disulfiram (ethanol) | Micon + Disulf (ethanol) | Ethanol |
|---|---|---|---|---|---|
| E cell NCTC 10418 | E ccl | >16 | >15 | >16 | >32 |
| E faecium NCTC 7171 | VanR Enterococcus | 12 | >16 | 4 | >32 |
| E gah, NCTC 12358 | VanR Enterococcus | 12 | >16 | 4 | >32 |
| EMRSA/L | MRSA | 4 | >16 | 2 | >32 |
| USA/VISA 5850 | VISA | 6 | 8 | 1 | >32 |
| S epidermidis NCTC 1228 | Sansithus Staphylocous | 2 | 12 | 1 | >32 |
| E faecim vanR B145344C LFE. | VanR Entotcocci | 12 | >16 | 4 | >32 |
| S suneus NCTC 6571 | Sanathus Staphyloccus | 6 | >16 | 4 | >32 |
| VISA 3900 UK | VISA | 4 | >t6 | 1 | >32 |
| E faecium VanA | VanR Entroccoti | 16 | >16 | 4 | >32 |
| MRSA LF 15 | MRSA | 4 | >16 | 2 | >32 |
| MRSA LF 18 | MRSA | 4 | >16 | 2 | >32 |

(continued)

| Strain | Type of strain | Miconazole | Disulfiram (ethanol) | Micon + Disulf (ethanol) | Ethanol |
|---|---|---|---|---|---|
| Mu50 JAPAN | VISA | 4 | =16 | t | >32 |

NB. Grey highlighting denotes a synergistic effect.

The results of the broth microdilation experiment (B) corroborate these findings, by demonstrating a clear synergistic effect against VISA strains. Synergy was observed for the combination of disulfiram with miconazole, econazole and clotrimazole (with the exception of strain VISA 3900 UK, against which clotrimazole and disulfiram did not demonstrate a synergistic effect).

**Table 2.** Broth microdilution demonstrating the synergistic effect of combining miconazole, econazole, or clotrimazole, with disulfiram.

| Strain | Miconazole | Miconazole + disulfiram | Econazole | Econazole + disulfiram | Clotrimazole | Clotrimazole + disulfiram | Growth control | Contamination control |
|---|---|---|---|---|---|---|---|---|
| Mu50exJapan (VISA) | 4 | 2 | 8 | 1 | 4 | 1 | OK | OK |
| S. epidermidis 1228 | 2 | 2 | 2 | 4 | 2 | 2 | OK | OK |
| VISA 3900 UK (VISA) | 2 | 1 | 8 | 4 | 4 | 4 | OK | OK |
| USA/VISA 5836 (VISA) | 8 | 1 | 8 | 1 | 8 | 1 | OK | OK |
| USA/VISA 5827 (VISA) | 4 | 1 | 4 | 1 | 2 | 1 | OK | OK |

NB. Grey highlighting denotes a synergistic effect. OK is reported in the control column if both the growth control shows good growth of the bacteria without the addition of any drugs, and the contamination control (which contains no bacteria) shows no growth, and therefore, no contamination.

**Table** 3

Table 3, below, shows the pooled results from the MIC testing of 25 different MRSA isolates and 5 VRE isolates. The MIC50 is the minimun inhibitory concentration required to inhibit the growth of 50% of the strains tested, and the MIC90, the concentration required to inhibit the growth of 90% of the strains tested. The MIC 50 and 90 for miconazole used singly, was 2mg/L and 2mg/L respectively, this was reduced to 0.5 and 1mg/L when 1mg/L of disulfiram was added to the miconazole.

**Table 3**. MIC 50, MIC 90 and range of MICs for the combination of miconazole and disulfiram.

| Drug Combination | MIC 50 | MIC 90 | Range | lowest MIC | highest MIC |
|---|---|---|---|---|---|
| Miconazole | 2 | 2 | 0 5-8 | 0 5 | 8 |
| 1,2 Micon Disulfiram | 1 | 1 | 0 5-4 | 0.5 | 4 |
| 2.1 Micon.Disulfiram | 1 | 2 | 1 - 2 | 1 | 2 |
| 1.1 Micon Disulfiram | 1 | 2 | 0.5 - 4 | 0.5 | 4 |
| Micon + 1mg/L Disulfiram | 0.5 | 1 | <0.25 - 2 | <0 25 | 2 |
| Micon + 2.5mg/L Disulfiram | 0.5 | 1 | <0.25 - 2 | <0 25 | 2 |

Table 3 shows that the MIC50, MIC90 and range of MICs obtained with miconazole against 25 MRSA strains and 5 VRE strains can be reduced by the addition of disulfiram. The best reduction was seen when a set amount of either 1 or 2.5mg/L of disulfiram was added, but significant reductions were also observed when a 1:1 ratio of miconazole to disulfiram was used.

## Claims

1. A composition comprising clotrimazole, or a salt thereof, and disulfiram, or a derivative thereof, for treating an infection caused by multi-drug resistant bacterial species.

2. A composition according to claim 1 wherein the derivative is a nitrate.

3. A composition according to any one of the preceding claims wherein the disulfiram derivative is a metabolite of disulfiram.

4. A composition according to any one of the preceding claims wherein the metabolite of disulfiram is selected from the group consisting of diethyldithiocarbamate and dimethyldithiocarbamate.

5. A composition according to any one of the preceding claims for prophylactic use.

6. A composition according to any one of the preceding claims wherein the multi-drug resistant bacterial species include MRSA, VISA, and/or VRSA.

7. A composition according to any one of the preceding claims wherein the ratio of clotrimazole, or a salt thereof, and disulfiram, or a derivative thereof, is 1:1.

8. A composition according to any one of the preceding claims wherein the synergistic combination comprising clotrimazole, or a salt thereof, with disulfiram and/or derivatives or metabolites thereof, is for administration topically to the site of an infection.

9. Clotrimazole, or a salt thereof, separately, simultaneously or sequentially in combination with disulfiram, or a derivative thereof, for treating an infection caused by multi-drug resistant bacterial species.

10. Clotrimazole, or a salt thereof, according to claim 9 for prophylactic use.

11. Disulfiram, or a derivative thereof, separately, simultaneously or sequentially in combination with clotrimazole, or a derivative thereof, for treating an infection caused by multi-drug resistant bacterial species.

12. Disulfiram according to claim 11 wherein the disulfiram is a metabolite of disulfiram.

13. Disulfiram according to claims 11 or 12 wherein the metabolite of disulfiram is selected from the group consisting of diethyldithiocarbamate and dimethyldithiocarbamate.

Europäisches Patentamt
European Patent Office
Office européen des brevets

# EUROPEAN SEARCH REPORT

Application Number
EP 12 00 2256

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BORISY A A ET AL: "SYSTEMATIC DISCOVERY OF MULTICOMPONENT THERAPEUTICS", PROCEEDINGS OF THE NATIONAL ACADEMY OF SCIENCES OF USA, NATIONAL ACADEMY OF SCIENCE, WASHINGTON, DC.; US, vol. 100, no. 13, 24 June 2003 (2003-06-24), pages 7977-7982, XP009061663, ISSN: 0027-8424<br>* page 7979; figure 1A *<br>----- | 1-13 | INV.<br>A61K31/415<br>A61K31/145<br>A61P31/04 |
| A | TAYLOR E H ET AL: "In vitro antimicrobial activity of diethyldithiocarbamate and dimethyldithiocarbamate against methicillin-resistant Staphylococcus", ANNALS OF CLINICAL AND LABORATORY SCIENCE, INSTITUTE FOR CLINICAL SCIENCE, PHILADELPHIA, PA, US, vol. 17, no. 3, 1 January 1987 (1987-01-01), pages 171-177, XP009115059, ISSN: 0091-7370<br>* abstract *<br>* page 174, left-hand column, paragraph 1 *<br>----- | 1-13 | TECHNICAL FIELDS SEARCHED (IPC)<br>A61K<br>A61P |
| A | PHILLIPS M ET AL: "Disulfiram inhibits the in vitro growth of methicillin-resistant Staphylococcus aureus", ANTIMICROBIAL AGENTS AND CHEMOTHERAPY 1991 US, vol. 35, no. 4, 1991, pages 785-787, XP002524036, ISSN: 0066-4804<br>* page 785, left-hand column, paragraphs 2,3 *<br>-----<br>-/-- | 1-13 | |

The present search report has been drawn up for all claims

1

| Place of search | Date of completion of the search | Examiner |
|---|---|---|
| Munich | 15 October 2012 | Escolar Blasco, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 00 2256

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | WO 2008/059258 A (THERAPEUTICS LTD E [GB]; YOUNG MALCOLM PHILIP [GB]; YATES CATHERINE MA) 22 May 2008 (2008-05-22)<br>* claims *<br>----- | 1-13 | |

TECHNICAL FIELDS SEARCHED (IPC)

The present search report has been drawn up for all claims

1

| Place of search | Date of completion of the search | Examiner |
|---|---|---|
| Munich | 15 October 2012 | Escolar Blasco, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 00 2256

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2012

| Patent document cited in search report | Publication date | Patent family member(s) | Publication date |
|---|---|---|---|
| WO 2008059258 A | 22-05-2008 | AU 2007320928 A1 | 22-05-2008 |
| | | CA 2669591 A1 | 22-05-2008 |
| | | EP 2094358 A2 | 02-09-2009 |
| | | EP 2508231 A2 | 10-10-2012 |
| | | JP 2010527323 A | 12-08-2010 |
| | | KR 20090100357 A | 23-09-2009 |
| | | US 2010144626 A1 | 10-06-2010 |
| | | WO 2008059258 A2 | 22-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2185187 A
- US 3249109 A
- US 53598122 B
- US 4144317 A
- US 4262003 A
- US 4307717 A